# EUROPEAN PATENT APPLICATION

(11) **EP 3 564 929 A1**
(43) Date of publication of application: **06.11.2019**
(21) Application number: 17885597.9
(22) Date of filing: 27.12.2017
(51) Int. Cl.: G09B 19/00, A61H 31/00, G01B 11/00, G09B 9/00

(54) **MEASUREMENT DEVICE AND PROGRAM**

(30) Priority: 27.12.2016 JP 2016254438
(71) Applicant: Coaido Inc., Tokyo 113-0033 (JP)
(72) Inventor: GENSHO, Makoto, Tokyo 113-0033 (JP); YAMADA, Yu, Tokyo 113-0033 (JP); OZAWA, Takahiro, Tokyo 113-0033 (JP)
(74) Representative: Bandpay & Greuter
(86) International application number: PCT/JP2017/046936
(87) International publication number: WO 2018/124188

(57) **Abstract**

To make it possible to perform measurement with a simple configuration. A measurement device 1 that is used for clinical practice or its training in cardiopulmonary resuscitation includes a storage unit 11 for storing therein the size of an AR marker attached at a prescribed position of a user, an imaging unit 12 provided with a visible light camera for capturing an image of the AR marker moving during measurement, a control unit 13 for detecting the position of the AR marker in the captured image and calculating the amount of displacement of the AR marker using the size of the AR marker stored in the storage unit 11, and an output unit for outputting the calculation result obtained by the control unit 13.

## Description

### Technical Field

An embodiment discussed herein relates to a measurement device and a program.

### Background Art

Computer-implemented methods for managing rescue training are known. For example, one of these methods involves registering a plurality of individuals as potential rescue trainees through a central computer server system, receiving, from a computing device that is remote from the central server system, information indicating the rate and depth of compressions performed by ones of the potential rescue trainees, and generating comparative data that reflects the performance of CPR chest compressions by a first rescue trainee for comparison with other rescue trainees. This method also involves providing the comparative data over a network for review to be made by one or more of the rescue trainees.

In addition, there is known a system that includes: a video camera provided in a position detector that is attached to a hand of a cardiac massage practitioner for capturing images of at least three markers; and a main device 2 connected to the video camera for displaying the movement of a measurement point representing the position of the position detector on the basis of changes in the positions of the markers captured by the video camera. This system has a measurement point determining means for determining the position of the measurement point, a measurement point tracking means for tracking changes in the position of the measurement point, and a display means for displaying the changes in the position of the measurement point on a monitor.

### Citation List

### Patent Literature

PTL1: Japanese Laid-open Patent Publication No. 2016-028290
PTL2: Japanese Laid-open Patent Publication No. 2013-153847

### Summary of Invention

### Technical Problem

In order to detect the positions of markers with infrared light, a dedicated device needs to be prepared, which increases the cost.

According to one aspect, the present invention is intended to enable measurement with a simple configuration.

### Solution to Problem

To achieve the above object, there is provided a disclosed measurement device. This measurement device is used for clinical practice or training for the clinical practice in cardiopulmonary resuscitation, and includes: a storage unit configured to store therein the size of a marker placed at a prescribed position of a user; an imaging unit provided with a visible light camera for capturing an image of the marker moving during measurement; a control unit configured to detect the position of the marker in the captured image and calculate an amount of displacement of the marker using the size of the marker stored in the storage unit; and an output unit configured to output a calculation result obtained by the control unit.

### Advantageous Effects of Invention

According to one aspect, it is possible to perform measurement with a simple configuration.

### Brief Description of Drawings

FIG. 1 illustrates a measurement system according to one embodiment.
FIG. 2 is a view for explaining an example of training.
FIG. 3 illustrates a hardware configuration of a measurement device according to the embodiment.
FIG. 4 is a block diagram illustrating functions of the measurement device according to the embodiment.
FIG. 5 is a view for explaining recognition of the types of plastic bottles.
FIG. 6 is a view for explaining a correspondence between plastic bottles and AR markers.
FIG. 7 is a view for explaining a screen to be displayed.
FIG. 8 is a view for explaining indicators.
FIG. 9 is a flowchart illustrating how the measurement device operates.
FIG. 10 is a view for explaining an example of a measurement result.
FIG. 11 is a view for explaining an example of training results.

### Description of Embodiment(s)

Hereinafter, a measurement system according to one embodiment will be described in detail with reference to the accompanying drawings.

### (Embodiment)

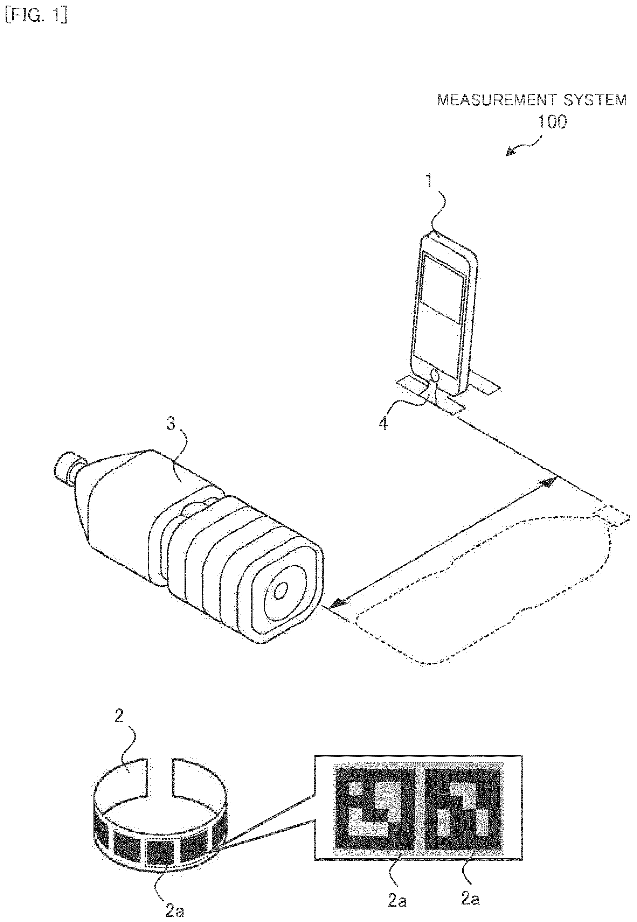

FIG. 1 illustrates a measurement system according to one embodiment.

The measurement system 100 of this embodiment enables clinical practice and its training in cardiopulmonary resuscitation (CPR), using a visible light camera provided in a measurement device 1.

The measurement system 100 of the first embodiment includes the measurement device (computer) 1, a wristband 2, and a plastic bottle 3

The measurement device 1 is provided with an imaging device, and is preferably a portable terminal, such as a smartphone or a tablet terminal, for example. In FIG. 1, the measurement device 1 is supported by a stand 4 so as to capture images of the top portion of the plastic bottle 3 lying on its side.

This measurement device 1 is provided with ArUco for marker detection. The ArUco is a lightweight AR (Augmented Reality) library that uses OpenCV (image processing library) released to the public under BSD license.

In measurement using this measurement device 1, a trainee wears the wristband 2 around his/her wrist. The wristband 2 is used for identifying movements of his/her hand, and has AR markers 2a printed on the front side thereof. In this connection, FIG. 1 illustrates black squares to indicate the locations of the AR markers 2a, but in actual each AR marker 2a has a different pattern as illustrated in a markup balloon.

The AR marker 2a is one of identifying means. Other identifying means include a bangle in which illuminators such as an LED (light emitting diode) are embedded, and a markerless method that uses a depth camera or the like to identify feature points.

Each AR marker 2a is square-shaped and, for example, is a square with sides of 15 mm to 20 mm. AR markers 2a of this size are arranged in a single line, so as to enable the measurement device 1 to complement measurement points. That is to say, use of one large AR marker may improve the accuracy of detecting this AR marker. However, if the measurement device 1 fails to detect the marker, it would be difficult to make a complement. By contrast, by arranging AR markers 2a of certain size in a single line, it is possible that, even if the measurement device fails to detect the measurement point of one AR marker 2a, the measurement device is able to detect the other AR markers 2a and add the measurement point as a complement.

In addition, FIG. 1 illustrates a case where the AR markers 2a are arranged in a single line, but they may be arranged in plural lines.

The plastic bottle 3 is used, instead of a human body, in training for cardiopulmonary resuscitation. By preparing different types of plastic bottles 3 and containing appropriately adjusted amounts of water in them, these plastic bottles 3 feel closer to human bodies in the training for the cardiopulmonary resuscitation. The types of plastic bottles to be prepared will be described in detail later.

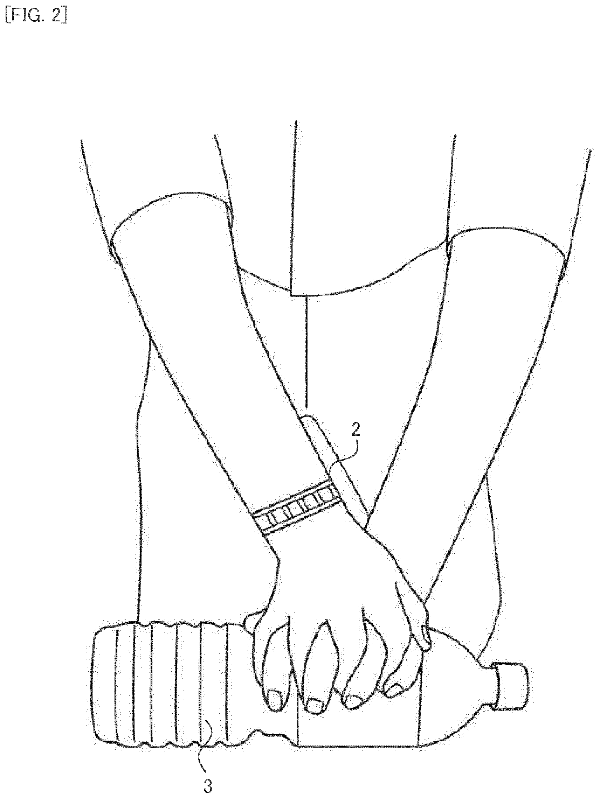

FIG. 2 is a view for explaining an example of training.

During training, a trainee is at such a position as to face the measurement device 1 and repeatedly compresses the plastic bottle 3 to an appropriate depth with an appropriate pressure (to be described later).

The measurement device 1 causes the above-described imaging device to capture a video image of an area surrounding the wristband 2 on the trainee's wrist. Then, the measurement device 1 analyzes the captured video image in real-time to detect the amount of movement, angle, movement rhythm, and others of his/her hand, in order to determine whether he/she is conducting the training properly.

Hereinafter, the disclosed measurement system will be described in more detail.

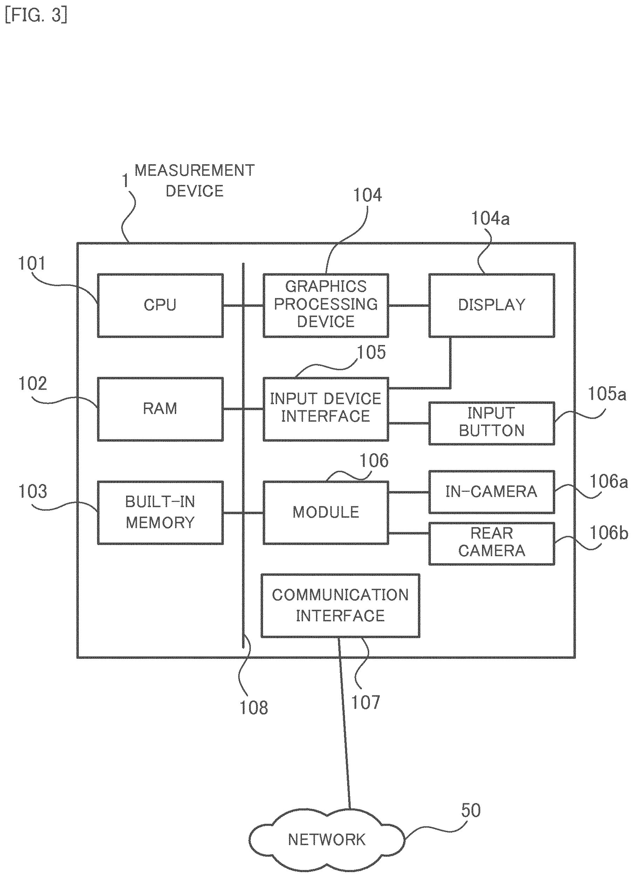

FIG. 3 illustrates a hardware configuration of the measurement device according to the embodiment.

The measurement device 1 is entirely controlled by a CPU (central processing unit) 101.

A RAM (random access memory) 102 and a plurality of peripheral devices are connected to the CPU 101 via a bus 108.

The RAM 102 is used as a main storage device of the mobile terminal 2. The RAM 102 temporarily stores therein at least part of OS (operating system) programs and application programs that are executed by the CPU 101. In addition, the RAM 102 stores therein various kinds of data to be used by the CPU 101 in processing.

Connected to the bus 108 are a built-in memory 103, a graphics processing device 104, an input device interface 105, a camera module 106, and a communication interface 107.

The built-in memory 103 performs data write and read. The built-in memory 103 is used as an auxiliary storage device of the mobile terminal 2. The OS programs, application programs, and various kinds of data are stored in the built-in memory 103. A semiconductor storage device, such as a flash memory, may be used as a built-in memory.

The graphics processing device 104 is connected to a display 104a. The graphics processing device 104 displays images on a screen of the display 104a in accordance with instructions from the CPU 101. Examples of the display 104a include a liquid crystal display. Also, the display 104a has a touch panel function.

The input device interface 105 is connected to the display 104a and an input button 105. The input device interface 105 gives signals received from the input button 105a and display 104a to the CPU 101.

The camera module 106 has an in-camera 106a that transmits visible light, on the front surface of the measurement device 1 (on a side closer to the display 104a), and a rear camera 106b on the back surface thereof. Images captured by the in-camera 106a and rear camera 106b are stored in the built-in memory 103 by the CPU 101 in accordance with user operation.

The communication interface 107 is connected to a network 50. The communication interface 107 communicates data with another computer or communication device over the network 50.

With the above hardware configuration, the processing functions of the present embodiment may be implemented.

The measurement device 1 having the hardware configuration illustrated in FIG. 3 is provided with the following functions.

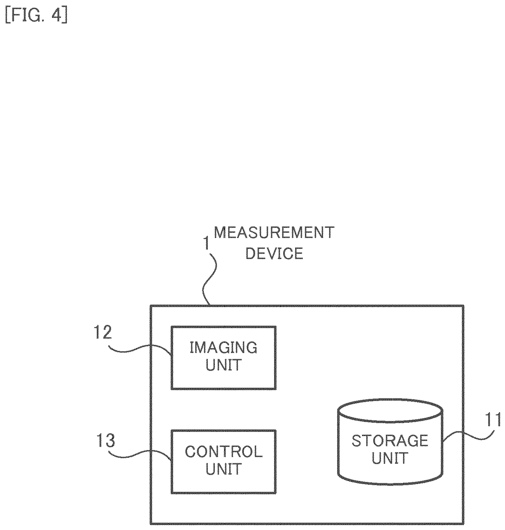

FIG. 4 is a block diagram illustrating the functions of the measurement device according to the embodiment.

The measurement device 1 includes a storage unit 11, an imaging unit 12, and a control unit 13.

The storage unit 11 stores herein a variety of programs used for measurement, moving images, and others. The storage unit 11 also stores therein the size of each AR marker 2a.

The imaging unit 12 causes the in-camera 106a or the rear camera 106b to capture a video image.

The control unit 13 entirely controls the measurement device 1. For example, the control unit 13 detects the position of an AR marker 2a in images captured by the imaging unit 12, and calculates the amount of displacement of the AR marker 2a using the size of the AR marker 2a stored in the storage unit 11.

The following describes a measurement method.

### (1) Recognize the type of a plastic bottle (preparation)

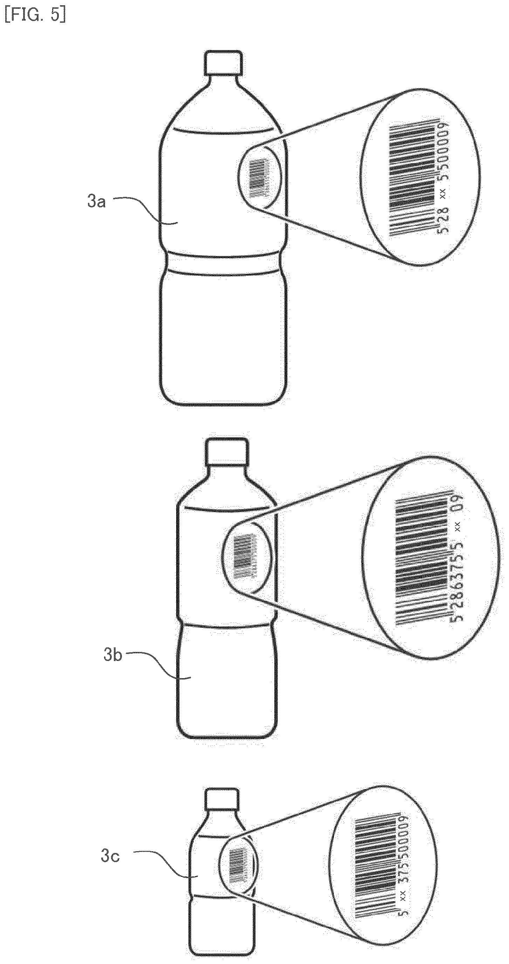

FIG. 5 is a view for explaining recognition of the types of plastic bottles.

Plastic bottles of different sizes and shapes are used as models of person who receives cardiopulmonary resuscitation. Before starting training, the type of a plastic bottle to be used is recognized to identify the plastic bottle as a model of person who has fallen.

Specifically, a trainee reads a barcode on the label of a plastic bottle 3 with the rear camera 106b of the measurement device 1. Barcodes are unique IDs and enable identifying the types (sizes) of plastic bottles 3a, 3b, and 3c easily. FIG. 5 illustrates plastic bottles 3a, 3b, and 3c of different sizes. The plastic bottle 3a has a capacity of 2 liters, the plastic bottle 3b has a capacity of 1 liter, and the plastic bottle 3c has a capacity of 500 milliliters. By reading the barcode, the trainee is able to recognize the type of the plastic bottle for preparation.

### (2) Display a lying person (who has fallen) in AR (preparation)

The plastic bottles 3 are used as AR markers.

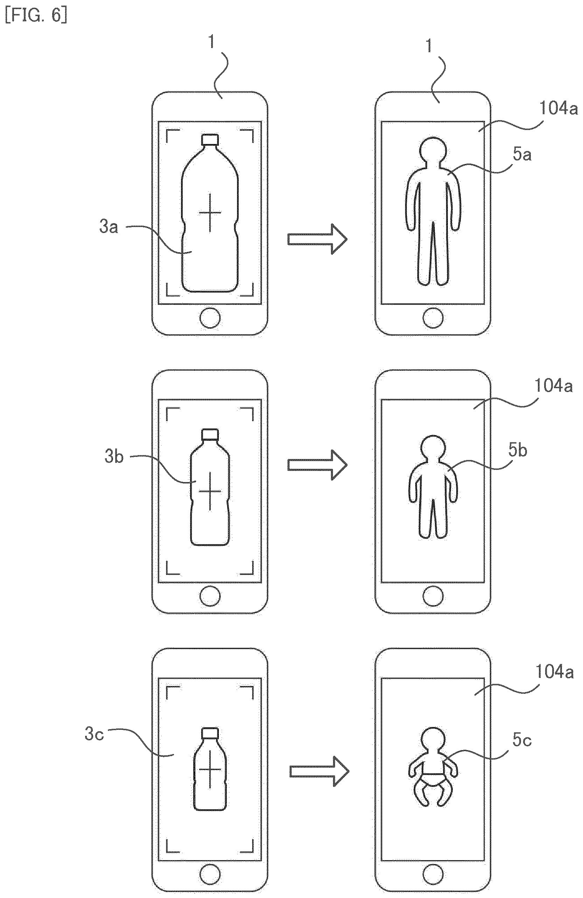

FIG. 6 is a view for explaining a correspondence between plastic bottles and AR markers.

The measurement device 1 displays an AR image of body shape and age based on the recognized shape of a plastic bottle in 3D. For example, when the imaging unit 12 captures an image of the plastic bottle 3a with a capacity of 2 liters, the control unit 13 displays an AR image 5a of an adult on the display 104a. The control unit 13 then gives a video guidance or the like on CPR training on an adult and the amount of water to be contained. The guidance on the CPR is useful for actual cardiac massages. When the imaging unit 12 captures an image of the plastic bottle 3b with a capacity of 1 liter, the control unit 13 displays an AR image 5b of a kid on the display 104a. In addition, the control unit 13 gives a video guidance or the like on CPR training on a kid and the amount of water to be contained. When the imaging unit 12 captures an image of the plastic bottle 3c with a capacity of 500 milliliters, the control unit 13 displays an AR image 5c of an infant. In addition, the control unit 13 gives a video guidance or the like on CPR training on an infant. In this connection, an AD image of a person of different gender, as well as different body shape and age, may be displayed in 3D according to the recognized shape of a plastic bottle. In addition, the above classification by size and shape of plastic bottle is just an example, and a 500-milliliter plastic bottle is not always used for CPR training on an infant.

Next, the trainee uses the stand 4 or the like to support the measurement device 1 vertically as illustrated in FIG. 1. Then, the trainee places the plastic bottle 3 at a location apart by about a distance corresponding to the height of a 2-liter plastic bottle from the measurement device 1. FIG. 1 illustrates a situation where the plastic bottle 3a is placed. To prevent the plastic bottle 3a from slipping, a non-slip sheet may be placed under the plastic bottle 3a or a rubber ring may be wounded around the plastic bottle 3a.

The trainee wears the wristband 2 on his/her wrist. Then, the trainee starts the measurement device 1 and activates an application. By doing so, the control unit 13 captures a video image of the plastic bottle 3a with the in-camera 106a.

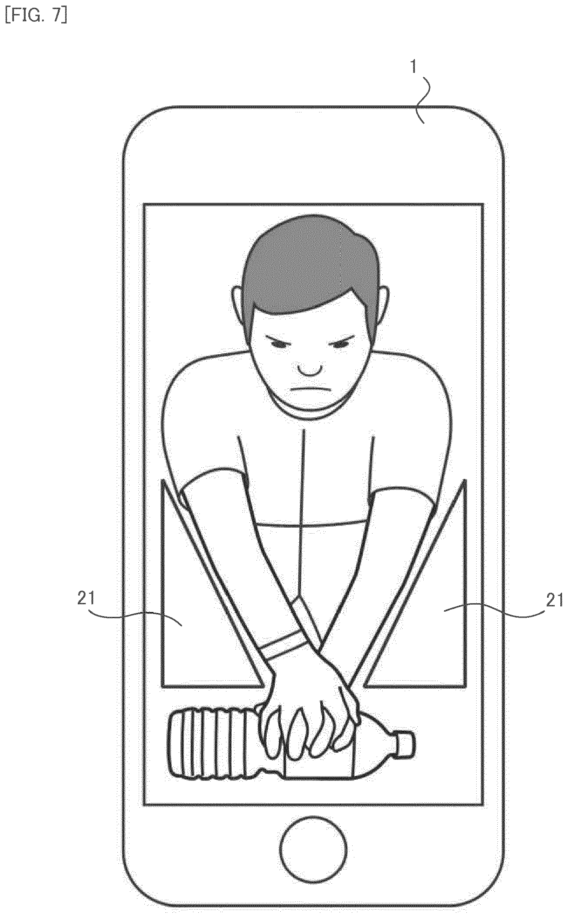

FIG. 7 is a view for explaining a screen to be displayed.

At this time, the control unit 13 displays a guide 21 for allowing the trainee to confirm the angles of his/her elbows on the display 104a. When the angles of his/her elbows match the guide 21, the control unit 13 starts measurement. In this connection, the control unit 13 may display another button to start the measurement on the display 104a.

After the measurement starts, the control unit 13 plays music to allow the trainee to take rhythms easily, and also displays, on the display 104a, two indicators indicating whether the training is proper. The type of music is not limited to any particular music, but if a guideline indicates that compressions of 100 to 120 times per minute are preferable, music that matches the guideline is preferable.

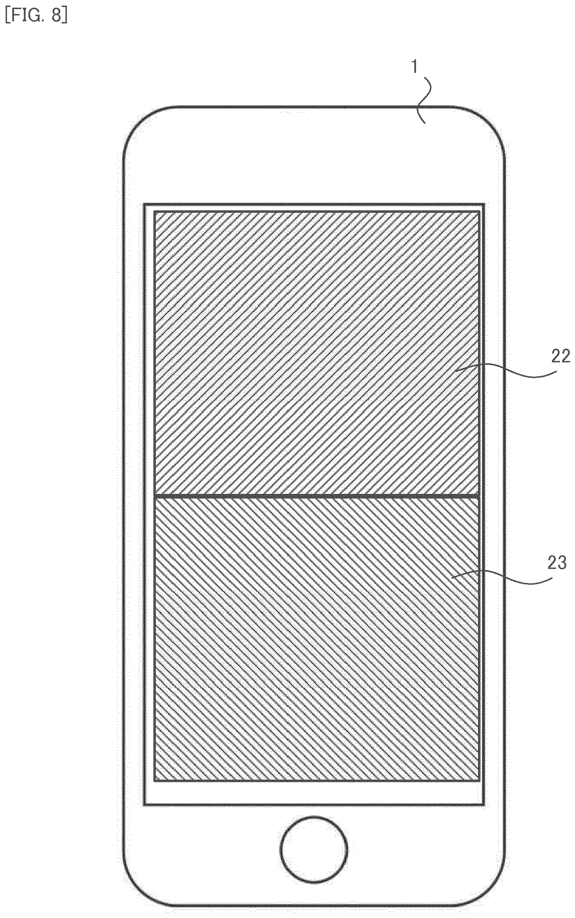

FIG. 8 is a view for explaining indicators.

An indicator 22 changes its color according to an amount indicated in a feedback from the control unit 13. More specifically, if the control unit 13 determines that the amount of recoil is less than or equal to 10 mm, the indicator 22 blinks in blue. This indicates that the training is going well, because the amount of recoil is 10 mm or less when a compression is fully released so that the plastic bottle 3a returns to the original position from a concave position after the plastic bottle 3a is compressed.

On the other hand, if the control unit 13 determines that the amount of recoil is greater than 10 mm, the indicator 22 blinks in red. This indicates that the training is not going well, because the amount of recoil is greater than 10 mm if a compression is not fully released.

An indicator 23 changes its color according to the amount of compression. More specifically, in the case where the plastic bottle 3a (representing an adult) is used, the indicator 23 blinks in blue if the amount of compression falls within 50 mm to 65 mm, whereas the indicator 23 blinks in red if the amount of compression does not fall within 50 mm to 65 mm. In the case where the plastic bottle 3b (representing a kid) is used, the indicator 23 blinks in blue if the amount of compression is one third of the thickness of the plastic bottle 3b, and blinks in red otherwise.

The control unit 13 may give the trainee an advice for correct compression via voice or the like if one or both of the indicators 22 and 23 blink in red. For example, when the indicator 22 blinks in red, the control unit 13 produces a voice saying "pull up more" or the like. When the indicator 23 blinks in red, the control unit 13 produces a voice saying "compress stronger" or the like.

The above feedbacks using the colors are just examples, and other colors, screens, blinking of light, sound, or others may be used.

In this connection, during the measurement, the control unit 13 may alternately display an image captured by the in-camera 106a, which photographs the trainee, and the indicators 22 and 23. Alternatively, during the measurement, the guide 21 may or may not be displayed in the images captured by the in-camera 106a.

In addition, the control unit 13 determines using the guide 21 whether trainee's elbows are bent, and may produce a voice depending on the determination result.

More specifically, the control unit 13 detects the inclination of the AR marker 2a. If the inclination of the AR marker 2a is greater than or equal to a prescribed value, the control unit 13 produces a voice saying "check if your elbows are bent" or the like. As another example, the control unit 13 may produce a voice saying "check if your elbows are bent" or the like if an arm overlies the guide or if an area where the arm and the guide overlap is greater than or equal to a prescribed size.

The following flowchart describes how the measurement device 1 operates.

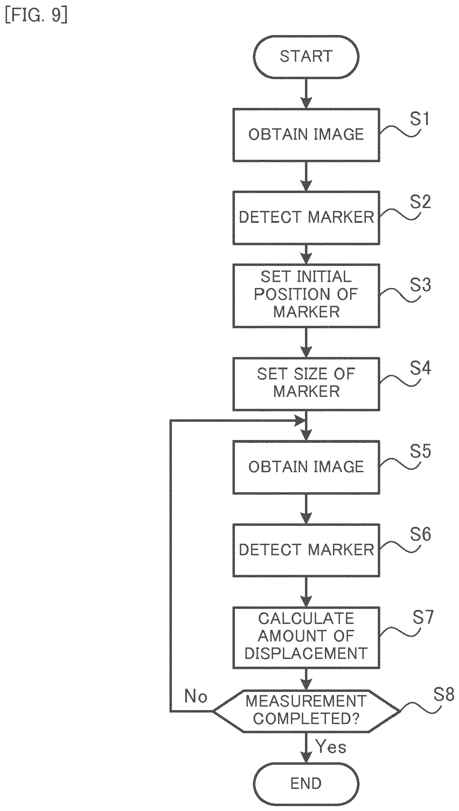

FIG. 9 is a flowchart illustrating how the measurement device operates.

First, the control unit 13 performs initialization. The control unit 13 performs the initialization in the first one frame, and repeats measurement until a trainee ends the application.

(Step S1) The control unit 13 obtains a still image from a video image captured by the in-camera 106a. Still images are not always obtained at fixed intervals. Then, the process proceeds to step S2.

(Step S2) The control unit 13 detects each AR marker 2a in the image with an ArUco library. By doing so, the marker identifier of each AR marker 2a and the coordinates of the four vertices of each AR marker 2a on the image are obtained. Then, the process proceeds to step S3.

(Step S3) The control unit 13 sets the coordinates of a vertex (for example, the coordinates of an upper left vertex) of each detected AR marker 2a as the initial position of the AR marker 2a. Then, the process proceeds to step S4.

(Step S4) The control unit 13 calculates the approximate size of each AR marker 2a on the image. More particularly, with respect to the Y coordinate of the four vertices of an AR marker 2a, the control unit 13 calculates the size of the AR marker 2a on the image by s = max("lower left vertex" - "upper left vertex", "lower right vertex" - "upper right vertex").

(Step S5) The control unit 13 obtains a still image from the video image captured by the in-camera 106a, as in step S1. Then, the process proceeds to step S6.

(Step S6) The control unit 13 detects the AR markers 2a in the image with the ArUco library, as in step S2. By doing so, the marker identifier of each AR marker 2a and the coordinates of the four vertices of each AR marker 2a on the image are obtained. In this connection, the control unit 13 enlarges a part of the image corresponding to each AR marker 2a and obtains the coordinates of the four vertices of each AR marker 2a on the image. Then, the process proceeds to step S7.

(Step S7) The control unit 13 calculates the amount of displacement of each AR marker 2a by subtracting its initial position from the newly detected position (upper left vertex) of the AR marker. The control unit 13 then calculates the actual amount of displacement by dividing it by the size of the AR marker 2a on the image. Then, the process proceeds to step S8.

(Step S8) The control unit 13 determines whether the trainee has completed the measurement. If the trainee has completed the measurement (Yes at step S8), the process of FIG. 9 is completed. If the trainee has not completed the measurement (No at step S8), the process proceeds to step S5.

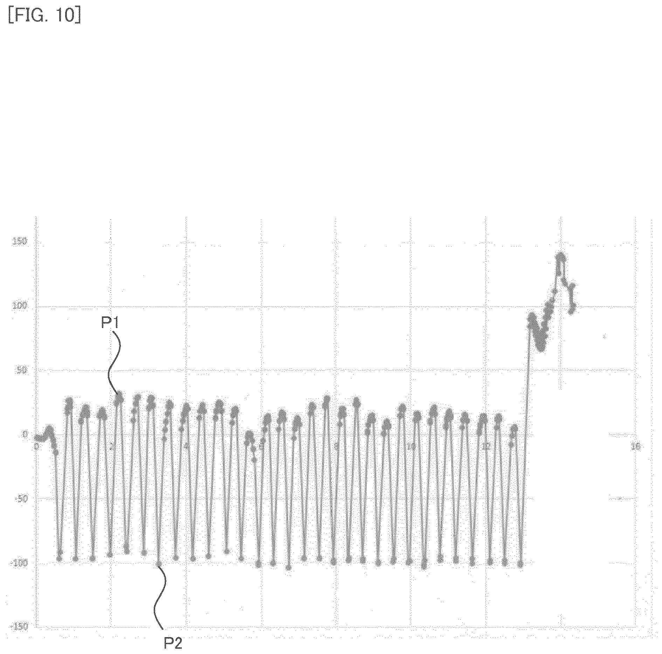

FIG. 10 is a view for explaining an example of a measurement result.

FIG. 10 illustrates a trajectory of one of a plurality of AR markers 2a detected by the control unit 13.

The vertical axis represents the depth of compression (moving distance of a hand), and the horizontal axis represents time. Points in the graph indicate the positions of the AR marker 2a detected by the control unit 13.

With the ArUco library, it is difficult to detect moving markers. This is because such moving markers may be blurred in images. However, theoretically, the speed at dead center points is zero in the CPR. Therefore, a marker may be detected at the dead center points. In an actual detection result, a top dead center point P1 is detected over several frames, and a sufficient accuracy is expected. A bottom dead center point P2 is detected in at least one frame.

The control unit 13 stores measurement results of the trainee in the storage unit 11.

Using the measurement results, the control unit 13 is able to calculate a variety of indices indicating a training result (whether the training has been conducted properly). For example, the control unit 13 is able to calculate a duty cycle (the time ratio of compression and decompression). In this connection, an ideal duty cycle is 50:50.

In addition, the control unit 13 is able to calculate the percentage of time in which chest compressions are performed (chest compression fraction (CCF)). An ideal CCF is in a range of 61% to 80%.

The control unit 13 creates a training result on the basis of these measurement results and calculation results. The control unit 13 outputs the created training result in CSV format or display it on the display 104a.

In this connection, FIG. 10 illustrates a trajectory of one AR marker 2a. Alternatively, the control unit 13 is able to create a training result by integrating results of detecting the plurality of AR markers 2a. For example, removal of noise based on the detection results of the AR markers 2a and complementing of dead points lead to an improvement in the accuracy of the created training result.

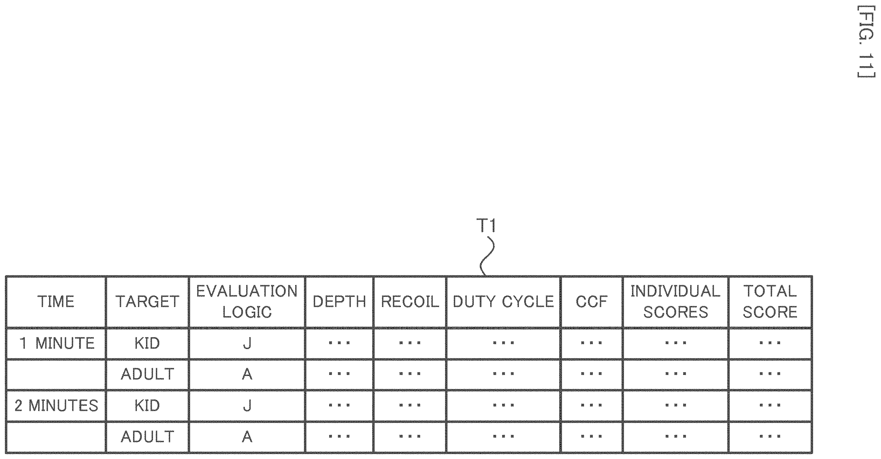

FIG. 11 is a view for explaining an example of training results. In FIG. 11, the training results are stored in tabular form.

The table T1 has the following columns: Time, Target, Evaluation Logic, Depth, Recoil, Rhythm, Duty Cycle (time ratio of compression and decompression), CCF, Individual Scores, and Total Score. The information items arranged in a horizontal direction are associated with each other.

The Time column contains a training time.

The Target column indicates a model used for training (any one from adult, kid, and infant).

The Evaluation Logic column contains an evaluation logic based on the type of the target.

The Depth column contains the result of evaluating a depth (OK or bad).

The Recoil column contains the result of evaluating recoil (OK or bad).

The Rhythm column contains the result of evaluating rhythm (OK or bad).

The Duty Cycle column contains the result of evaluating a duty cycle (OK or bad).

The CCF column contains the result of evaluating CCF (OK or bad).

The Individual Scores column contains a score indicating a successful rate with respect to the individual items including depth, recoil, rhythm, duty cycle, and CCF.

The Total Score column contains a total score.

In addition, scores are compared among a plurality of trainees, and their placements in ranking or ranking (within town, prefecture, or country) may be displayed.

In this connection, a result of determining using the guide 21 whether elbows are bent may be reflected on a training result. A score is decreased if the elbows are detected to be bent.

In this connection, the embodiment uses the plastic bottles 3 as training targets. The above training method may be applied to cardiopulmonary resuscitation for a human body. That is, in principle, it is possible to utilize the above measurement when cardiopulmonary resuscitation is performed on a human body, not on a doll or a plastic bottle. This is usable for proper cardiopulmonary resuscitation in not only training but also clinical practice.

As described above, the measurement device 1 stores the sizes of the AR markers 2a obtained with the visible light camera, in the storage unit 11, and calculates the amount of displacement in each marker to obtain a training result. Therefore, it is possible to perform training with a simple device configuration.

In addition, a plurality of AR markers 2a are provided, and the control unit 13 complements the positions of AR markers using a result of detecting the AR markers. This achieves an improvement in the accuracy of a created training result.

In addition, the control unit 13 displays an AR image of a person of different age and gender according to the features of the plastic bottle 3 on the display 104a. Thereby, the trainee is able to image a training target easily.

In addition, the control unit 13 determines using the guide 21 whether trainee's elbows are bent, and reflects the determination result on the training or a training result. Therefore, it is possible to make use of the training for clinical practice.

In addition, the control unit 13 enlarges a part corresponding to an AR marker 2a in an image captured by the in-camera 106a, and then calculates the amount of displacement in the AR marker 2a. This improves the processing speed.

In this connection, the processing performed by the measurement device 1 may be performed by a plurality of devices in a distributed manner. For example, one device may calculate the amount of displacement of an AR marker 2a and another device may output a training result using the amount of displacement.

In addition, when the international consensus and guideline for cardiopulmonary resuscitation are updated, the indices of the measurement and the measurement logic are updated accordingly.

Heretofore, the measurement device and program of the embodiment have been described with reference to the embodiment illustrated. The invention is not limited thereto, and the components of each unit may be replaced with other components having equivalent functions. In addition, other desired configurations and steps may be added to the invention.

Further, two or more desired configurations (features) in the above-described embodiment may be combined.

The above-described processing functions may be implemented by using a computer. In this case, a program is prepared, which describes the processing contents of the functions of the measurement device 1. A computer implements the above-described processing functions by executing the program. The program describing the intended processing contents may be recorded on a computer-readable recording medium. Computer-readable recording media include magnetic storage devices, optical discs, magneto-optical recording media, semiconductor memories, etc. The magnetic storage devices include hard disk drives, flexible disks (FDs), magnetic tapes, etc. The optical discs include DVDs, DVD-RAMs, CD-ROMs, CD-RWs, etc. The magneto-optical recording media include MOs (magneto-optical disk), etc.

To distribute the program, portable recording media, such as DVDs and CD-ROMs, on which the program is recorded may be put on sale, for example. Alternatively, the program may be stored in the storage device of a server computer and may be transferred from the server computer to other computers over a network.

A computer that is to run the above program stores in its local storage device the program recorded on a portable recording medium or transferred from the server computer, for example. Then, the computer reads the program from the local storage device, and runs the program. The computer may run the program directly from the portable recording medium. Also, while receiving the program being transferred from the server computer over a network, the computer may sequentially run this program.

In addition, the above-described processing functions may also be implemented wholly or partly by using DSP (digital signal processor), ASIC (application-specific integrated circuit), PLD (programmable logic device), or other electronic circuits.

### Reference Signs List

1 Measurement device
2 Wristband
2a AR marker
3, 3a, 3b, and 3c Plastic bottle
4 Stand
5a, 5b, and 5c AR image
11 Storage unit
12 Imaging unit
13 Control unit
21 Guide
22, 23 Indicator
100 Measurement system

## Claims

1. A measurement device to be used for clinical practice or training for the clinical practice in cardiopulmonary resuscitation, the measurement device comprising:
a storage unit configured to store therein a size of a marker placed at a prescribed position of a user;
an imaging unit provided with a visible light camera for capturing an image of the marker moving during measurement;
a control unit configured to detect a position of the marker in the captured image, and calculate an amount of displacement of the marker, using the size of the marker stored in the storage unit; and
an output unit configured to output a result of the calculation obtained by the control unit.

2. The measurement device according to claim 1, wherein at training, the control unit displays a target of different body shape, age, and gender according to features of a plastic bottle.

3. The measurement device according to claim 1 or 2, wherein during the measurement, the control unit determines whether an elbow of the user is bent, and outputs a result of the determination.

4. The measurement device according to any one of claims 1 to 3, wherein the control unit enlarges a part of the image corresponding to the marker, and calculates the amount of displacement of the marker, the image being captured by the visible light camera.

5. A computer program to be used for clinical practice or training for the clinical practice in cardiopulmonary resuscitation, wherein the computer program causes a computer to perform a process including:
detecting a position of a marker in an image captured by a visible light camera that captures the image of the marker moving during measurement;
calculating an amount of displacement of the marker, using a size of the marker placed at a prescribed position of a trainee, the size being stored in a storage unit; and
outputting a result of the calculating.
